(19) Europäisches Patentamt
European Patent Office
Office européen des brevets

(11) EP 0 831 769 B2

(12) NEW EUROPEAN PATENT SPECIFICATION
After opposition procedure

(45) Date of publication and mention of the opposition decision:
**23.07.2008 Bulletin 2008/30**

(45) Mention of the grant of the patent:
**15.10.2003 Bulletin 2003/42**

(21) Application number: **96924268.4**

(22) Date of filing: **07.06.1996**

(51) Int Cl.:
***A61K 8/44*** *(2006.01)* ***A61Q 19/08*** *(2006.01)*
***A61Q 17/04*** *(2006.01)* ***A61Q 19/00*** *(2006.01)*

(86) International application number:
**PCT/US1996/009975**

(87) International publication number:
**WO 1996/040048 (19.12.1996 Gazette 1996/55)**

# (54) NOVEL USES FOR THYROID HORMONES OR THYROID HORMONE-LIKE COMPOUNDS

NEUE VERWENDUNGEN VON THYROIDHORMONEN ODER THYROIDHORMON AEHNLICHE VERBINDUNGEN

UTILISATIONS INNOVANTES D’HORMONES THYROIDIENNES OU DE COMPOSES RESSEMBLANT A DES HORMONES THYROIDIENNES

(84) Designated Contracting States:
**AT BE CH DE DK ES FI FR GB GR IE IT LI LU MC NL PT SE**

(30) Priority: **07.06.1995 US 481698**

(43) Date of publication of application:
**01.04.1998 Bulletin 1998/14**

(60) Divisional application:
**03022417.4 / 1 398 024**

(73) Proprietor: **Lavin, Dr., Thomas N.**
**Watchung, NJ 07069 (US)**

(72) Inventors:
- **LAVIN, Thomas, N.**
**Watchung, NJ 07060 (US)**
- **VAHLQUIST, Anders, B.**
**Institutionern for Dermato.**
**S-581 85 Linköping (SE)**

(74) Representative: **Austin, Hedley William et al**
**Chapman Molony**
**Cardiff Business Technology Centre**
**Senghennydd Road**
**Cardiff CF24 4AY (GB)**

(56) References cited:

| | |
|---|---|
| **EP-A- 0 060 776** | **WO-A-01/76589** |
| **BE-A- 515 812** | **CH-A- 642 851** |
| **FR-A- 2 139 748** | **FR-A- 2 153 202** |
| **FR-A- 2 354 101** | **FR-A- 2 357 246** |
| **FR-M- 6 656** | **GB-A- 782 745** |
| **GB-A- 859 546** | **GB-A- 1 354 263** |
| **US-A- 3 198 702** | |

- **STN, File Supplier, Karlsruhe, DE, XP002020415**
- **STN, File Supplier, Karlsruhe, DE, XP002020416**
- **STN, File Supplier, Karlsruhe, DE, XP002020417**
- **STN, File Supplier, Karlsruhe, DE, XP002020418**
- **DATABASE WPI Week 8205 Derwent Publications Ltd., London, GB; AN 82-004847 XP002020419 & RO 76 691 A (INTR. FARMEC. COSMETI.) cited in the application**
- **DE RYCKER ET AL.: "Effects of 3,5,3’-triiodothyronine on collagen synthesis by cultured human skin fibroblasts" FEBS LETTERS, vol. 174, no. 1, August 1984, ELSEVIER SCIENCE PUBLISHERS, pages 34-37, XP002041073 cited in the application**
- **STN, File Supplier, Karlsruhe, DE, File XP002040739**
- **STN, File Supplier, Karlsruhe, DE, File XP002040740**
- **STN, File Supplier, Karlsruhe, DE, File XP002040741**
- **STN, File Supplier, Karlsruhe, DE, File XP002040742**
- **STN, File Supplier, Karlsruhe, DE, File XP002040743**
- **Dictionnaire Vidal,Paris,O.V.P.1990.Editorial pages(3) and pages 1774-1775 (Triacana crème).**
- **Thyroid,13,2,159-165,2003**
- **J.Lab.Clin.Med.,69,950-9,1967**
- **Endocrinology, 80,1051,1967**
- **Thyroid,11,717-724,2001**



Printed by Jouve, 75001 PARIS (FR)

## Description

### TECHNICAL FIELD

This invention relates to skin care using thyroid hormones, thyroid hormone metabolites, derivatives of those compounds and other thyroid hormone receptor binding chemical entities to improve the appearance of the skin.

### BACKGROUND

A wide variety of skin care preparations are currently available. There are currently available several preparations for the treatment of cellulite, a dimpling of the skin over excess superficial fat deposits, but these are believed to have doubtful efficacy. A wide variety of medically useful skin preparations are also currently available, comprising primarily glucocorticoids and retinoid topical medicaments, both of which have varying side-effects and usefulness. There is a considerable number of skin conditions and diseases such as stria, cellulite, roughened skin, actinic skin damage, intrinsically aged skin, photodamaged skin, lichen planus, ichtyosis, acne, psoriasis, wrinkled skin, eczema, seborrhoeic dermatitis, scleroderma, hyperkeratinizing disorders, keloids and skin scarring.

The structurally similar thyroid hormones triiodothyronine ($T_3$) and thyroxine ($T_4$) have a very wide range of effects. In adult mammals they influence nearly all organs, the metabolism of nutrients, basal metabolic rate and oxygen consumption. In humans, the deficiency or excess of circulating thyroid hormones results in the well characterised syndromes, hypo- and hyperthyroidism. Small concentrations of thyroid hormone metabolites which are also endocrinologically active exist. Among these compounds are tri-iodothyroacetic acid("Triac") and tri-iodoproprionic acid ("Tri-prop").

Thyroid hormones exert many of their actions by binding to a family of receptor proteins termed the *C-erb-A* family. In humans, this receptor protein family is now known to comprise several members, notably the human thyroid receptor alpha-1, the human thyroid receptor alpha -2 which binds the hormone poorly or not at all, the human thyroid receptor β-1, and the human thyroid receptor β-2. These proteins are part of a larger superfamily of steroid hormone receptors which comprises the glucocorticoid receptors, the retinoic acid receptors, the vitamin D receptors, and the insect moulting receptors - the receptors for ecdysone and the insect juvenile hormones. Receptors for thyroid hormones are found in human skin, human fibroblasts and keratinocytes and they are also found in all other tissues within the human body (1).

In addition to the naturally occurring thyroid hormones, a large number of chemical compounds which bind to the thyroid hormone receptor and which produce thyroid hormone-like effects have been synthesized see for example US5401772.

Thyroid hormones, in many cases, act indirectly by influencing the effects of other hormones and tissues (1). For example in the rat, thyroid administration increases pituitary growth hormone production which in turn affects hepatic protein production including that of alpha-2 euglobulin. Functionally, in the rat, growth hormone may act as a second message for thyroid hormone(1). The biology of thyroid hormones has been extensively studied after oral administration, which makes the relationship between a direct effect of thyroid hormones and an indirect effect mediated by thyroid hormone modulation of other autocrine, paracrine or endocrine factors difficult to ascertain.

Orally administered thyroid hormones influence the connective tissue biology of the skin. When given orally, thyroid hormones induce an increase in neutral salt and acid soluble collagen, but decrease insoluble collagen in the skin of guinea pigs (2). Fibronectin production is decreased in human fibroblasts and fibroblast glycosoaminoglycans are either decreased or unchanged depending on the experimental conditions used (3,4,5,7,7a). Keratin gene expression for both the basal cell keratin K5 and K14 genes and the differentiation specific K10 gene is negatively regulated by thyroid hormones (9,8) in keratinocyte culture. These effects are mirrored by similar cell culture responses to retinoic acid (9) or the retinoid Tretinoin (19).

Histological studies of skin from individuals who have an excess of thyroid hormone show an increased number of cell layers in the skin, reflected by mean epidermal cell number, increased protein turnover with increased proline incorporation and generalized increases in epidermal proliferation compared to normal skin (11). In individuals who have a lack of thyroid hormone, the skin is atrophic with thinning of the epidermis and a decrease in cellularity. In human clinical biology, thyroid hormone excess leads to a general smoothing of the skin and the loss of wrinkles especially over the olecranon (elbow) surface.

Thyroid hormones also accelerate fat synthesis and lipolysis (breakdown). In rats which have an excess of thyroid hormone either chemically or by natural means, fat stores are in general decreased (12,13), although in humans clinical observation of thyroid hormone excess discloses either increase or decrease in weight. The synthesis of fats may be increased (14,15) or decreased (12,13,16,21). Attempts to utilize the effects of excess oral thyroid hormone for weight loss in humans have in general failed because of severe adverse side effects (25,26).

Orally given thyroid hormone compound or thyroid hormone-like compounds in excess of normal bodily requirements or medical conditions which are associated with excess thyroid hormone such as Grave's disease or toxic

nodular goitre produce an acceleration of heart beat with associated heart failure, cardiac arrhythmias, osteoporosis, increased intestinal motility leading to diarrhoea, psychiatric abnormalities, and an increase in the basal metabolic rate. Attempts to use oral thyroid hormones for diminishing lipid levels in man resulted in increased cardiac deaths (17).

RO76691 and 76692 respectively disclose an anti-wrinkle cream comprising a crude preparation from animal endocrine glands, including the thyroid gland, and a method of obtaining lipoid extracts from animal byproducts respectively. No data on the efficacy of the anti-wrinkle cream is provided nor is the thyroid hormone content of the cream provided or even mentioned. In particular, there is no suggestion in those patents of the efficacy of the compositions, methods, and uses of the present invention.

The use of Triac and its salts for a reduction of cellulite is disclosed in FR 2.153.202 (7134447). FR2197577 (72.30781) discloses various derivatives of Triac, including para hydroxy esters thereof, as having utility for the same indication. EP060776 discloses activity of an isopropyl derivative of Triac for the same indication. CH642851 (1168/80) discloses the utility of a liposome formulation of Triac together with glycosoaminoglycans for reducing cellulite. GB1354263 also discloses the use of Triac itself for reducing fat deposits. GB1400851 relates to the synthesis of ethyl esters and alkyl carboxy acids derivatives of Triac and their use in reducing cellulite in combination with leeches, hyaluronidase, proteases, and lipase.

None of the above publications disclose or suggest the usefulness of compounds selected from those that bind the thyroid hormone receptor. The relationship between Triac and thyroid hormones is not made in any of these patents and no data is presented to show the relationship of the above mentioned compounds to the thyroid hormone dependent endocrine system via an effect mediated through the human thyroid receptor. In fact, FR2354101 discloses the use of Triac as an inhibitor of phosphodiesterase and its effect to increase cyclic AMP. This effect occurs only at dose ranges of approximately $10^{-4}$ M in *in vitro* experiments using isolated adipocytes. In concert with this mode of activity, the prior art literature makes claims for effective concentrations of chemical entity for anti- cellulite effects as greater than 50 mg of Triac or Triac derivative per 100 ml of vehicle and usually between 100 and 200 mg /100 gms of excipient.

FR96.171 discloses the topical cellular growth accelerating activity and mitogenic of thyroxine (0.001-0.008 mg. %) in a propylene glycol diethyl ether vehicle in rat skin. No mention is made of other compounds which have thyroid hormone receptor binding activity such as Triac or Tri-iodothyronine, or other thyromimetic compounds known at the time (Journal of Medicinal Chemistry, 6, p 554-563, 1963). No teaching concerning the effects of thyroid hormone receptor binding entities on epidermal differentiation, gene expression or keratinization is made. GB782,745 and 859,546 describe the topical usefulness of compounds of a class which partially overlap with chemical entities which bind to the thyroid hormone receptor, but includes compounds including L-T-1 with no thyroid hormone receptor binding activity, and only a small subset of compounds which do recognize the receptor. In these patents, no claims are made for chemical entities characterized by binding to the thyroid hormone receptor or having thyroid hormone like activity. US3198702 discloses a method of treating burns comprising the topical application of certain thyroxin analogues which are a subset of the same class of compounds claimed to have activity in GB 782745 and 859546.

No prior art document discloses the skin differentiating and collagen promoting effects of thyroid hormone or its analogues.

## SUMMARY OF THE INVENTION

According to one aspect of the invention there is provided a method of cosmetic treatment of skin using Triac, the method having the features defined in claim 1.

According to another aspect of the invention there is provided the use of Triac for the manufacture of a topical skin treatment medicament according to claim 10.

According to a further aspect of the invention there is provided a method of cosmetic treatment of skin using either triiodothyronine ($T_3$) or a thyroid hormone-like compound, the method having the features defined in claim 11.

The method according to the invention improves the appearance of skin without causing the undue adverse effects of orally administered thyroid hormone in a direct thyroid hormone dependent manner avoiding renal and hepatic metabolism of the thyroid hormone receptor binding chemical entity. In particular, the method of delivery of the triiodothyronine and thyroid hormone-like compounds avoids liver and kidney metabolism of the hormones, blood circulation of the hormones to other tissues and binding to blood carrier proteins which can alter efficacy.

For the purposes of this invention a "thyroid hormone-like compound" is any chemical entity, including peptides, which at least partially binds to thyroid hormone receptor TR-$\alpha$ or $\beta$ with a chemical affinity constant, $K_d$, lower than 1$\mu$M when tested in receptor binding assay, described by Lavin (27) using pure or substantially pure natural or recombinant thyroid hormone $\alpha$ or $\beta$ receptor containing the ligand binding domain or thyroid hormone receptor containing preparations such as rat nuclei . Such ligands may be considered hormones when they have similar agonistic effects as the natural hormone or may be considered antagonists when the compounds antagonize the effects of the natural hormones. Partial agonist/antagonists also may exist. (Suitable ligands may be agonists or antagonists).

The topically-applied triiodothyronine or thyroid hormone-like compounds used according to the present invention are advantageous in that they enable the use of these chemical compounds to improve the appearance of the skin, without causing the undue adverse effects of orally administered thyroid hormone. In particular, the method of delivery of the triiodothyronine and thyroid hormone-like compounds avoids liver and kidney metabolism of the hormones, blood circulation of the hormones to other tissues and binding to blood carrier proteins which can alter efficacy.

The triiodothyronine or thyroid hormone-like compound is preferably in pure form, i.e not contaminated with other similar compounds.

The thyroid hormone-like compound may be selected from, for example, one of the following illustrative list of compounds:

3,3',5' triiodothyronine (reverse $T_3$) ; 3,3'-diiodothyronine ; $T_3T_4$ analogues such as 3,5,3'- Triiodothyroacetic acid ; 3,5,3'-Tetraiodothyroacetic acid ; 3,5,3'-Triiodo-L-thyronine ; 3,5,3'-Triiodo-L-thyronine methyl ester; 3,5,3'-Triiodo-L-thyronine hydrochloride; L-$T_3$;$T_4$Fo ; Tetrac; Triac; Tetraprop ; Triprop ; $T_4$Bu ; $T_3$Bu ; Thyroxamine ; Triiodothyronamine ; L-3'-$T_1$; L-3,5'- $T_2$ ; L-3,3'-$T_2$; L-3,3',5'-$T_3$; DL-$Br_2$I; L-$Br_2$iPr ; L-$Me_2$I; L-$Me_3$; L-$Me_4$; L-$Me_2$iPr DL-IMeI ; 1-3,5- Dimethyl-3'-isopropylthyronine (DIMIT) ; DL-$BPT_4$ ; B-triac ; BP- tetrac; DL-$SBT_3$; DL- $SBT_4$ ; DL-$MBT_3$ ; MB-tetrac ; $T_2$ ; $T_2$F ; $T_2$Cl; $T_2$Br; $T_3$; $T_2$Me ; $T_2$Et; $T_2$iPr ; $T_2$nPr ; $T_2$SBu ; $T_2$tBu ; $T_2$iBu ; $T_2$Phe ; $T_2$OH ; $T_2NO_2$; $T_2F_2$ ; $T_2Cl_2$ ; $T_4$ ; $T_2Me_2$; 3,5,3'-Triiodo-D-thyronine; 3,5- Diiodo-4- hydroxyphenylpropionic acid (DIHPA); Aryloxamic acids ; (arylamino) acetic acids ; arylpropionic acids ; arylthioacetic acids ; (aryloxy)acetic acid ; 3,3'-$T_2$; 3,5-$T_2$ ; 3'-5'-$T_2$ ; (5-Benzyloxy-2-methoxyphenyl)-(2-methoxypyrimidin-5-yl)-methanol ; Benzyloxy-2- methoxyphenyl)-(6-methylpyridin-3-yl)methanol ; (5-Benzyloxy-2- methoxyphenyl)-(5-bromo-2-methoxypyridin-4-yl)methanol ; (5-benzyloxy-2-methoxyphenyl)-(2,6- difluoropyridin-3-yl)methanol ; (5-Benzyloxy-2-methoxyphenyl)- (2-methoxypyridin-4- yl)methanol; 4-Methoxy-3-[(2-methoxypyrimidin-5-yl)methyl]phenol ; 4-Methoxy-3-[(6-methylpyrid-3-yl) methyl]phenol; 5-Benzyloxy-2- methoxybenzyl Bromide ; (5- Benzyloxy-2-methozyphenyl)-(6-chloropyridazin-3-yl)-acetonitrile; 4-Benzyloxy-2-[2- methoxythiazol-5-yl)methyl]anisole ; 6-[(5-Hydroxy-2-methoxyphenyl)methyl]thiazol-2- (3H); 3'-Heteroarylmethyl-4'-)-methyl-3, 5-dinitro-N- trifluoro-acetyl-L-thyronine Ethyl Esters; 3'-heteroarylmethyl-3,5-di-iodo-4')-methyl-N-trifluoro-acetyl-L-thyronine Ethyl Esters ; 3'-heteroarylmethyl analogues of 3,3',5-tri- iodo-L-thyronine ($T_3$) ; 3'-substituted derivatives of the thyroid hormone 3,3',5-triiodo-L-thyronine (T3); $\alpha$ -methyl-3,5,3'-triiodothyroacetic acid, $\alpha$-methyl-3,5,3'-triiodothyropropionic acid, and $\alpha$-methyl-3,5,3'5'-tetraiodothyropropionic acid; methylene- and carbonyl-bridged analogs of iodinated thyronines or thyroacetic acids; or Iodinated benzofurans; 3,5-diiodo-4-(2-N,N-diethylaminoethoxy)phenyl-(2-butylbenzofur-3-yl)methanol hydrochloride; 2-n-butyl-3-(3,5-diiodo-4-carboxymethoxy-benzoyl)benzofuran;
2-methyl-3-(3,5-diiodo-4-carboxymethoxy-benzyl)benzofuran; [4'-hydroxy-3'-iodo-3, 5 diido-4-(2-N,N-dimethylamino-(ethoxy)benzophenon hydrochloride;
2-butyl-3-(3-iodo-4-hydroxybenzoyl)benzofuran; 4'4-dihydroxy 3'3,5-triiodo-diphenylmethane; 3,5-diiodo-4-(2-N, N-diethylaminoethoxy)phenyl-;
-(2-butylbenzofur-3-yl)methanol hydrochloride; 2-methyl-3-(3,5-diiodo-4-(2-N, N-diethylamino-ethoxy)-benzoyl) benzofuran hydrochloride; 2-n-butyl-3-(3,5-diiodo-4-carboxymethoxy-benzoyl)benzofuran;
2-methyl-3-(3,5-diiodo-4-hydroxy-benzoyl)benzofuran;
2-methyl-3-(3,5-diiodo-4-carboxymethoxy- benzyl)benzofuran;
4'hydroxy-3'-iodo-3,5 diiodo-4-(2-N,N-dimethylamino- ethoxy)benzophenon hydrochloride ; 2-butyl-3-(3-iodo-4-hydroxybenzoyl)benzofuran ;
3,5-diethyl,3'-isopropyl thyronine (DIET); 4',4-dihydroxy-3'3,5-triiododiphenylmethan ; and IpTA2 (3,5 diiodo- 3'isopropyl thyroacetic acid) and

pharmacologically acceptable salts and derivatives, such as metabolites, and effective isomers thereof.

Other suitable thyroid hormone-like compounds, are disclosed for example in US5284971, US5401772, US3649679, US3357887, US412579, US4168385, US5179097, EP0580550, EP018351 and H. A. Selenkow and S. P. Asper. Jr., Physiol. Rev. 35 426 (1955); C. S. Pitman and J. A. Pitman In Handbook of Physiology, Section 7: Endocrinology, Vol. 3, R. O. Greep and E. B. Astwood. Eds., Thyroid American Physiological Society, Washington D.C., 1974, p. 233; E. C. Jorgensen, Pharm. Ther. B, 2, 661 (1976); and E. C. Jorgensen, 'Thyroid Hormones and Analogs. **II.** Structure-Activity Relationships," in Hormonal Proteins and Peptides, Vol. 6. Thyroid Hormones, C. H. Li, Ed., Academic, New York, 1978, p. 108. The choice of other suitable thyroid hormone-like compounds for use in the compositions and methods of the present invention is within the scope of the skilled worker.

Preferably, the composition is in the form of a cream although other forms of preparation which can be readily applied to the skin, such as lotions, topical sprays, liposomes, solutions or emulsions, are contemplated. Preferably the composition includes suitable epidermal penetration enhancing agents.

The composition comprises less than about 50mg/100ml, more preferably less than about 10mg/100ml of the triiodothyronine or thyroid hormone-like compound. Preferably the composition comprises a concentration 5 x $10^8$ times or less the receptor dissociation constant, $K_d$ of the triiodothyronine or thyroid hormone-like compound. Preferably the

composition is used to supply an effective amount of the triiodothyronine or thyroid hormone-like compound which generally ranges from 500mg/m$^2$ to 0.1mg/m$^2$ in one or more applications, preferably 250mg/m$^2$ to 1mg/m$^{2,}$ per day in one or more applications.

The effective concentration will depend on factors such as metabolism of the compound and its effective octanol/water partition coefficient and which can be readily determined by the skilled addressee. The composition used according to the invention may also include other ingredients such as Vitamin D, glucocorticoids and retinoids or analogues thereof to potentiate and modify the effects of the triiodothyronine or thyroid hormone-like compound for increased benefit. The composition may also include BHT (butylated hydroxy toluene) or BHA (butylated hydroxy anisole) as a hindered phenol to decrease iodine decomposition or substances. Furthermore, the composition may include compounds which facilitate passage of the thyroid hormone through the skin and compounds which act as sunscreens such as PABA. Preferably, the composition also includes a suitable antioxidant such as Tinuvin P or BHA. The choice of such compounds is within the scope of the skilled addressee. See for example Hermens W. A. J. J Pharmaceutisch Weekblad Scientific Edition 14(4A) 1992.

The pharmacologically acceptable base is preferably an oil in water emulsion or an alcoholic solution with glycerol.

The said at least one thyroid hormone-like compound is preferably at least partially dissolved in a solvent. The solvent is preferably an organic solvent selected from alcohol and alcohol and water solutions. More preferably, the organic solvent is selected from isopropanol, isopropanol and water, ethanol, and ethanol and water solutions containing at least 20% alcohol.

Preferably, the composition is applied from twice a day to every three days.

## DETAILED DESCRIPTION OF THE INVENTION

Compositions for use in accordance with the invention and methods for their use will now be described, by way of example only, of with reference to the accompanying drawings, Figure no. 1-5.

## BRIEF DESCRIPTION OF THE DRAWINGS

Fig. 1 is a graph showing the comparative effect of betamethasone and Triac on the Skin-2 model;

Fig. 2 is a graph showing the comparative effect of vitamin D3 and Triac on the Skin-2 model;

Fig. 3 is a graph showing the comparative effect of retinoic acid and Triac on the Skin-2 model;

Fig. 4 is a graph showing the effect of retinoic acid on the skin-2 model in three studies;

Fig. 5 is a graph showing the effect of Triac on the skin-2 model in three studies.

EXAMPLE 1

*IN VITRO* TESTS WITH A SKIN A MODEL

Regulation of epidermal genes which regulate collagen production and promote epidermal differentiation.

Terminal differentiation of the epidermal layer of the skin is thought to be related at least in part to the activity of tranglutaminase K and the production of cornified envelopes (Febs Letter 258 p35-38, 1989, and J. Invest . Derm. 90 p472). It is thought that the ability of a retimoid to down regulate transglutaminase K in cultured cells is an indication of its ability to effectively treat psoriasis (EP00465343). TGF beta 1 and 2 are thought to promote terminal differentiation and inhibit basal cell proliferaton (Fuchs E. J. Cell Biol. 111 p2807). A protease, stratum corneum chymotryptic enzyme, SCCE, is produced by keratinocytes and is thought to promote shedding of skin cells in the upper granular layer of the epidermis and be related to epidermal differentiation (Egulrud et al, ACTA DERM Venereol. Vol 73 p 181 -184 1993: Sondell et al, J. Invest Derm 1995, p 819-823). Further, in hyperproliferating skin, the keratins 1 and 10 (differentiation markers) are reduced and the hyperproliferation keratins 16 and 6 are increased ( West et al 1992, J. Invest. Derm.1 p 95). Retinoic acid effects on skin keratinocytes in culture reflect a decrease in the differentiation markers and variable changes in keratins 6 and 16 and they also decrease transglutaminase *K in vitro.* Both these markers, the keratins and tranglutaminase K have been considered in-vitro measures of retinoic acid analogs dermatologic potency and usefullness (Kopan and Fuchs, J.Cell Biol 105 p 427-440, 1987 and Griffiths et al, Br J Derm 1992, 127 suppl 4 p 21. Paradoxically,

however, retinoids are active against hyperproliferating disorders and increase the keratin 1 and 10 differentiation markers and tranglutaminase K *in vivo* after topical application. (Griffiths et al, Br. J. Derm 1992, 127 suppl 4 p21. In keratinocyte cultures, thyroid hormone decreases keratin genes K5, K14 and K10 (6,10). Thyroid hormone modulation of the keratin 1 gene has not been previously reported.

Collagen production in the skin occurs from a variety of collagen genes, with the procollagen A-1 and A-3 genes most studied. Collagen is decreased in glucocorticoid treated skin (7) and in photodamaged skin (24). It is thought that overproduction of collagen in the hyperproliferative state may contribute to keloidal scar production. Furthermore, CTGF, a PDGF like peptide (PDGF-L) that is produced in response to TGF beta-1 appears to be present in increased amounts in fibrosing disorders such as schleroderma. In cultured human fibroblasts, thyroid hormone administration decreases collagen production (de Rycker et al, FEBS Lett, vol 174 p34-37 1984).

No universally accepted model of human skin exists. A novel synthetic human skin, Skin- 2 model ZK1301 (Advanced Tissue Sciences, La Jolla (USA)) is composed of a three dimensional human skin culture consisting of a dermal fibroblast layer and human foreskin keratinocytes which can be grown with an air-keratinocyte interface to promote normal differentiation and cornification of the model skin. The Skin-2 model is widely accepted as an in-vitro model to asess cutaneous toxicity (Rheins et al, Toxic, in Vitro, 8, no. 5 p1007-1014 1994 and is approved by the Canadian and U.S. FDA as an appropriate model for testing skin toxicity. Such a model has the following advantages:

1) The keratinocytes differentiate in a manner very similar to the *in vivo* state. A stratified multilayered epithelium with all the markers and properties of such epithelium forms. This never happens in typical keratinocyte culture.
2) The presence of fibroblasts allows the detection of fibroblast markers including collagen. The interplay between keratinocytes and fibroblasts which occurs *in vivo* also is mimicked in the $SKIN^2$ model, which allows paracrine functioning of the cell types.We assessed the ability of multiple thyroid hormone receptor binding ligands, and several therapeutically useful model compounds including retinoic acid to alter mRNA production of a variety of genes present in fibroblasts and keratinocytes present in the Skin-2 model.

**Materials and Methods**

The following were components of the $Skin^2$ kits: 9x9 mm skin tissues on agarose, 6-well plates, and Millicell inserts. Two media based on Dulbecco's Modification of Eagle's medium were also included:
Maintenance medium (containing 5% fetal calf serum) and serum-free assay medium.

The following were supplied by other sources: "Stripped" (Samuels, H. et al, Endocrinology 105, p80-85, 1979) fetal calf serum and all substances to be tested were from Karo Bio AB. All-trans-retinoic acid,
1,25-(OH)2-vitamin D3, and betamethasone were purchased from Sigma Chemicals (Stockholm). Upon arrival the skin tissues were placed dermal-side down into the center of a Millicell in a 6-well plate well containing prewarmed maintenance medium. The tissues were incubated at 37°C in an atmosphere of 5% $CO_2$ in air. After 24 h the maintenance medium was exchanged.

Following another 24 h the Maintenance medium was removed and replaced by Serum- free assay medium containing 5% "stripped" fetal calf serum. Substances were added within 1 h after medium exchange and the tissues were incubated for 10 or 48 h in the presence of the substances. Medium and substances were changed after 24 h incubation. The cells were grown at an air - liquid interface. At the end of the 48 h-incubation period two tissues were pooled together in a sterile plastic centrifugation tube. The tissue was snap frozen on solid ice (-60°C) and stored at -70°C until used. Duplicate samples were included for each treatment.

**Analysis of mRNA expression in $Skin^2$ by reversed-transcriptase coupled to polymerase chain reaction (RT-PCR)**

Total RNA was extracted from $Skin^2$ -samples and human skin biopsies using Ultraspec II (Biotecx Laboratories, Inc., Houston, TX). One ml of Ultraspec II was added to each sample which were homogenized by using a Polytron for 2x20 sec at setting 6. Total RNA was extracted according to the protocol supplied by the manufacturer. The amount of RNA was quantitated by measuring the absorbance at 260 nm. The A260/A280 -ratio of the extracted RNA was generally between 1.75 and 2.0. Three μg RNA was reversed transcribed into cDNA in a 30 ul mixture containing 50 mM Tris-HC (pH 8.3), 75 mM KC, 3 mM MgC2, 10 mM dithiothreitol, 0.5 mM of each dNTP, 200 U M-MLV reversed transcriptase (Life Technologies), 25 U placental Rnase inhibitor (Boehringer-Mannheim) and oligo d(T)15 as primer. After incubation at 37°C for 60 min, the reaction was stopped by heating at 75°C for 10 min and the mixture stored at -70°C until cDNA amplification was performed.

**Polymerase chain reation**

The following PCR mixture (24 μl) was prepared immediately before use: 10 mM Tris-HC (pH 8.3), 50 mM KC, 1.5 mM MgCl, 200 μM of each dNTP, 0.5 μM primers (see Table 1), and 0.5 U Taq DNA polymerase licensed for PCR (Life Technologies). The tubes were placed in a Thermal cycler (Perkin-Elmer) programmed as follows: (a) 90°C for 90 s; (b) variable number of cycles (see Table 1) of the following sequential steps: 60 s at 94°C, 75 s at annealing temp (see Table 1), 90 s at 72°C; and (c) 7 min at 72°C.
After mixing 7.5 μl of PCR-products with 2 μl of loading buffer, samples were separated by electrophoresis on a 1.2 % agarose gel containing ethidium bromide, and visualized by UV transillumination. Gels were recorded by videoprints. The amount of product was estimated on the videopoint or directly on the gel. The amount of transcripts in each lane were compared in relation to two control samples run in parallel.
The expression of certain genes was not modulated by test compounds, and so these genes were not selected as markers in subsequent experiments

Table 1

| Gene | Annealing Temp | PCR-cycles | Product Size (bp) |
|---|---|---|---|
| **Fibronectin** | **60°C** | **22-25** | 794.0000000 |
| IL-6 | 60°C | 22-25 | 639.0000000 |
| IL-8 | 60°C | 25.0000000 | 275.0000000 |
| ICAM-1 | 60°C | 30.0000000 | 727.0000000 |
| TGF β1 | 60°C | 25.0000000 | 338.0000000 |
| TGF β2 | 56°C | 40.0000000 | 575.0000000 |
| Keratin 1 | 60°C | 24-27 | 480.0000000 |
| Keratin 10 | 60°C | 27-30 | 439.0000000 |
| Keratin 5 | 60°C | 25.0000000 | 559.0000000 |
| Keratin 14 | 60°C | 25.0000000 | 651.0000000 |
| Keratin 6 | 60°C | 25.0000000 | 464.0000000 |
| Keratin 19 | 60°C | 20.0000000 | 400.0000000 |
| Keratin 16 | 60°C | 22-25 | 725.0000000 |
| SCCE | 60°C | 22.0000000 | 463.0000000 |
| Collagenase | 60°C | 30.0000000 | 463.0000000 |
| Involucrin | 60°C | 25.0000000 | 541.0000000 |
| Filaggrin | 60°C | 29.0000000 | 526.0000000 |
| Loricrin | 60°C | 33.0000000 | 663.0000000 |
| Procollagen 1A1 | 60°C | 20-22 | 620.0000000 |
| Procollagen 1A2 | 60°C | 23.0000000 | 712.0000000 |
| Procollagen 3A1 | 60°C | 20-24 | 524.0000000 |
| TGk | 60°C | 22-30 | 652.0000000 |
| CTGF | 60°C | 25.0000000 | 515.0000000 |
| ThR θ | 60°C | 32.0000000 | 623.0000000 |
| ThR β | 60°C | 32.0000000 | 578.0000000 |
| CRBPI | 60°C | 25.0000000 | 388.0000000 |
| CRABPII | 60°C | 22-25 | 402.0000000 |
| RAR θ | 60°C | 35.0000000 | 817.0000000 |

(continued)

| Gene | Annealing Temp | PCR-cycles | Product Size (bp) |
|---|---|---|---|
| RAR β | 60°C | 35.0000000 | 769.0000000 |
| RAR | 60°C | 30.0000000 | 765.0000000 |
| RXR θ | 60°C | 30.0000000 | 743.0000000 |
| VDR | 60°C | 30.0000000 | 888.0000000 |
| cyclophilin | 60°C | 20-22 | 424.0000000 |
| GAPDH | 60°C | 24-27 | 893.0000000 |

Key

IL-6 interleukin-6, IL-8 interleukin-8, ICAM-1 intracellular adhesion molecule-1, TGF β transforming growth factor, SCCE stratum corneum chymotryptic enzyme, $TG_k$ keratinocyte transglutaminase, CTGF connective tissue growth factor, ThR thyroid hormone receptor, CRBP cellular retinol-binding protein CRABP II cellular retinoic acid- binding protein, RAR retinoic acid receptor, RXR retinoid X receptor (9-cis-RA receptor), VDR vitamin $D_3$ receptor, GAPDH glyceraldehyde-3-phosphate dehydrogenase.

The effect of tri-iodothyroacetic acid (Triac), was first studied and compared to known medically useful compounds which affect epidermal physiology. These drugs were retinoic acid, betamethasone, and 1,25 OH vitamin $D_{-3}$. The semi quantitative results are shown in Table 2 and comparisons between Triac and the other drugs are displayed in fig. 1, (betamethasone), Fig. 2 (Vit D3) and Fig. 3 (all trans Retinoic acid). Triac at 50 nMolar concentration, approximately 1000 times its affinity constant for the receptor, appears to strikingly reduce keratin 1 and 10, tranglutaminase K, and SCCE. Triac also markedly accentuates Collagen A-1 and 3A-1, along with TGF-beta-2 transcripts. In comparison with betamethasone, Triac increases collagen production and TGF beta-2 but does not strongly inhibit IL-6. The keratin findings are similar. In comparison with Triac, Vit D-3 does not inhibit the keratin gene transcripts, or SCCE or transglutaminase K., but the effect on collagen genes is similar. Retinoic acid and Triac appear very similar with the exception of only SCCE and CRAB PII. At a higher concentration, 1μMolar, Triac continues to display the same effects although the colagen and keratin response is somewhat reduced. It is a surprising finding of the present invention that low doses of thyroid hormone compounds are dermatologically effective.

These results are consistent with known results of these drugs in other model systems. For example Vitamin D-3 is not known to affect keratinization of cultured keratinocytes. Betamethasone decreases epidermal collagen. Retinoic acid is known to decrese transglutaminase Ki and keratins 1 and 10 in culture. These results therefore suggest that Triac should have similar properties as these other medically important classes of drugs. In fact a combination of a thyroid hormone with a glucocorticoid should avoid the collagen loss associated with topical steroid application and add an antiinflammatory effect not seen with a thyroid compound.

The results of three experiments with Triac at 50 nM dose and retinoic acid at 1 μMolar dose are shown for comparison in figures 4 and 5. The two hormones appear very similar, but can be differentiated by the lack of inhibition of SCCE by RA in this model, and the modest fall in transcripts for the CRAB PII retinoid binding protein induced by Triac. Both compounds inhibit keratin 1 and more modestly, keratin 10, increase TGF-beta-2, CTGF, collagen and inhibit transglutaminase K.

A group of thyroid hormone receptor binding compounds were therefore tested in this model. Eight different compounds which span a range of different structures, including nonhalogenated compounds and compounds containing bromine instead of iodine, all of which bind to the receptor were assayed in the Skin-2 system. One compound, L-T-1, as described in GB7822745, GB859546 and US3198702 as part of a family of molecules which have topical utility, which bind very poorly to the receptor was also tested. The compounds were all tested at concentration ranges above their binding affinity for the receptor except for L-T-1 which binds poorly to the receptor even at the tested ten micromolar concentration. Table 3 displays the results for these compounds along with their $K_i$ (binding affinity) to the human thyroid receptor and the tested concentration of chemical. Affinities for the human thyroid receptor alpha-1 were similar. Again, a reduction in transcripts for keratin 1 (8/8 compounds), keratin 10 (5/8), and transglutaminase K (4/8) was seen, along with increased collagen a-1 (7/8) and collagen 3a (6/8) and CTGF (7/8). For the most part, an inhibition of SCCE was seen, although in some instances a very modest change was displayed by some compounds. The compound (L-T-I), which did not bind to the endogenous receptor, showed no inhibition of keratin 1 or 10, a positive effect on transglutaminase K and SCCE which is opposite to the thyroid response and retinoic acid response, and only modest effects on collagen.

Certain of the compounds, notably compound number 5 displayed a relative selectively for keratin 1, tranglutaminase K and SCCE responses over collagen responses. Therefore it seems likely that some of the compounds

will display a relative efficacy more weighted toward regulation of epidermal differentiation than collagen production and potential would healing effects or amelioration of dermal states characterized by collagen deficient skin.

Although the effects of these thyroid compounds as a group reflect specific and reproducible changes in the transcripts of epidermally related genes which affect differentiation, the *in vitro* direction of the response for SCCE, Transglutaminase K and keratin 1 is the opposite to that which might be considered therapeutically useful. Since a similar result is seen for retinoic acid (Koppam *et al;* Griffiths et *al* supra), these two entities are therefore quite similar in their effects. Surprisingly the collagen response to thyroid hormone was the opposite to that reported in the literature (de Ryk *et al supra).* Because of the paradoxically opposite effects of retinoids on skin related proteins and genes when given in vitro as compared to *in vivo* testing, and the concomitant medically beneficial usefullness of retinoids. It was considered that thyroid regulation would be subject to the same paradox. therefore Triac was tested on human volunteers.

## EXAMPLE 2

### HUMAN STUDY: THYROID HORMONE CONTROLS GENE TRANSCRIPTION IN SKIN

Three adult male volunteers ranging in age from 37-46 yrs were treated topically with a cream containing either 200 mg/100 ml or 20 mg/100 ml Triac. The cream was applied in an amount sufficient to just cover an approximately 6 $cm^2$ area on the buttocks. A similar sized area on the contralateral side, treated with a dermatologically useful cream, served as control. After application, the areas were covered with Tegaderm (3M) to prevent loss of cream and to enhance penetration. Control and Triac-treated areas were biopsied 96 h following this single treatment. The degree of erythema of each subject was judged on a subjective scale 1 to 4. Epidermis and papillary dermis from control and treated skin were obtained by manually cutting superficially with a razor-blade following 1 % lidoacine anesthesia. Two pieces of tissue measuring 2 x 2 cm and approximately 0.2 mm in depth were obtained for RNA-preparation. The tissue was snap frozen on solid ice (-60°C) and stored at -70°C until used.

A large number of transcripts were tested in three male subjects and the results are shown in table 4. The change in transcription of the genes for SCCE, Keratin 1 and 10, and transglutaminase K was now unexpectedly positive, the opposite of what was found in the *in vitro* assay. These changes reflect an increase in the differentiation markers and suggest a change to a more terminally differentiated state. The effect on collagen and CTGF remains the same *in vivo* as *in vitro* when using the Skin-2 model. Thus, at least for gene products which are related to epidermal differentiation the thyroid response *in vitro* is opposite to that *in vivo,* an identical finding as has been seen for the medically important retinoid compounds. Moreover, the unexpected increase in collagen formation suggest utility to help rebuild photodamaged adult human skin (Talwar et al, J. Inv. Derm, 105 p285-290, 1995).

## EXAMPLE 3

### THYROID HORMONES ARE PHYSIOLOGICALLY ACTIVE WHEN APPLIED TO THE SKIN.

Triac (Tri iodo thyroid acetic acid) or DIMIT (dimethyl isopropyl thyronine) or esters and ethers thereof are mixed in a concentration range of 10 000 000 times $K_{diss}$ to 1000 times $K_{diss}$,100 micromolar to 1 nanomolar, in a suitable pharmacological base, such as Johnson and Johnson Purpose™ or Curity™ skin lotion and applied to hairless mice strain SKH/hr 1 for 8 weeks. Punch biopsies are obtained and ultrasound measurements were made of the skin using a 10 MHz B-mode ultrasound (18). As compared to control animals, thyroid hormone treated animals have an increase in the thickness of the epidermal area and a slight decrease in the dermal portion of the skin. An increased cellularity of epidermal cell layers is found, with a decrease in the regularity of the rete pattern.

## EXAMPLE 4

### PHOTODAMAGED SKIN

Hairless mice SKH-1 are UV-B irradiated according to the schedule of (22). After 12 weeks of irradiation, significant wrinkling results. The mice are treated with the thyroid hormone Triac-containing cream for a period of 16 weeks after irradiation. The degree of wrinkling appearing on the irradiated mice is compared to untreated, irradiated animals to which the appropriate pharmaceutical base is not applied. The thyroid hormone containing composition of the invention produces a significant decrease in the wrinkle pattern of the animals and an increase in skin collagen.

The composition used in the present invention may be conveniently supplied in a unit dose pack comprising a plastic container which may for example be bubble, or blow moulded or injected, together with a tear off plastic or foil top, the pack containing a unit dose of the composition.

**REFERENCES**

1. Lavin, T.N. Mechanisms of Thyroid Hormone action. In the textbook of Endocrinology (DeGroot, Ed.), 2nd Edition, W. B. Saunders, pub. (1989).
2. Drozdzm M et al. Endokrinologie 1979 Feb; 73(1)105-11
3. Murata Y et al J Clin Endocrinol Metab 1987 Feb; 64(2): 334-9
4. Watxke, H., et al Thrombosis Research. 46(2): 347-53, 1987 Apr 15
5. Murata Y et al JCEM 1983 Vol. 57 p. 1233-1239
6. Lee J et al Endocrinology 130 p 2733-2738, 1992
7. Ceccarelli, P et al JCEM 65 p 242-246, 1987
8. Tomio-Canic M et al Journal of Investigative Dermatology 99 p 842-847, 1992
9. Blumenberg M et al. J. Invest Dermatol 1992 Jun: 98(6 Suppl):42S-49S
10. Ohtsuki M et al J Dermatol 1992 Nov 19(11) p 774-80
11. Holt, P.J.A et al British Journal of Dermatology 95 p 513-518, 1976
12. Cronrath CM et al Endocrinology, 120(1) page 43-8, 1987 Jan
13. Towle H. C, Mariash C. N Federation Proceedings 45(9) p 2406-11, 1986 Aug.
14. Lemmen C. et al Biological Chemistry Hoppe-Seyler 367(7) p 533-537, 1986
15. Amorosa L. F et al Biochim Biophys Acta 1984 Feb 9, 792(2) p 192-8
16. Armer P et al Diabetes 33(4) p 369-375, 1984
17. Coronary Dru Project, JAMA 220 p 996-1008, 1972
18. Akesson, A et al Acta Radiologica Diagnosis 27 p 91-94, 12986
19. Hunt, T et al Annals Surgery 170 p 633-641, 1969
20. Varani, J et al J. Invest Dermatol 94 p 717-723, 1990
21. Loireau A et al Biochem Pharmacol 35 p 1691-1696, 1986
22. Moloney S. J et al Photochem Photobiol 56 p 505-511, 1992
23. Schwartz E et al J. lnvestl. Dermatol 102 p 241 -246,1994
24. Griffiths C. E. M et al NEJM 329 p 530-535, 1993
25. Roti, E et al Endocrine Reviews 14 p 401-423, 1993
26. Lev-Ran A Perspectives in Biology & Medicine 37 p 486-494, 1994
27. Apriletti J. et al J. Biol. Chem. 263 p 9409-9417, 1988


TABLE 2

COMPARISON OF TRIAC WITH OTHER DERMATOLOGICALLY ACTIVE DRUGS

Genes Tested

| drug | k1 | k10 | k16 | k19 | transgK | scce | tgfbeta-2 | IL-6 | col 1A-1 | col 3A-1 | ctgf (pdgf-L) | crabp-II |
|---|---|---|---|---|---|---|---|---|---|---|---|---|
| vit D 1 μMolar | 0 | -1 | 0 | 0 | -1 | 0 | 2 | -1 | 2 | 2 | 0 | -1 |
| Triac 50 nM | -3 | -2 | -1 | -1 | -3 | -2 | 2 | -0.5 | 2 | 2 | 1 | -1 |
| Triac 1 μM | -2 | -2 | -0.5 | -1 | -3 | -2 | 2 | -0.5 | 2 | 1 | 0.5 | -1 |
| betameth 1 μM | -3 | -0.5 | -1 | -0.5 | -1 | -1 | 0 | -2 | 0.5 | -0.5 | 1 | -1 |
| RA 1 μM | -3 | -1 | -0.5 | -1 | -3 | -0.5 | 2 | -1 | 2 | 1 | 1 | 1 |

TABLE 3

TRANSCRIPTIONAL RESPONSES TO THYROID HORMONE RECEPTOR BINDING ENTITIES 1μMOLAR CONCENTRATION

| | compound number | Genes tested | | | | | | | |
|---|---|---|---|---|---|---|---|---|---|
| Ki (nM) | | k1 | k10 | k16 | trans gk | scce | col a-1 | col a-3 | gf(pdgf-L) |
| 0.1 | 1 | -2 | -0.5 | 0 | 0 | 0.5 | 1 | 2 | 1 |
| 0.1 | 2 | -1 | 0 | 0 | 0.5 | 0.5 | 2 | 1 | 1 |
| 10 | 3 | -2 | -1 | 0 | 1 | 0 | 2 | 0.5 | 1 |
| 20 | 4 | -2 | -0.5 | 0 | 0 | 0 | 1 | 0 | 1 |
| 20000 | L-T-1 10uM | 0 | 0 | 0 | 1 | 1 | 0.5 | 0.5 | 1 |
| 0.04 | Triac 50nM | -2 | -2 | | 0 | 0 | 2 | 1 | 2 |
| | Triac 1 uM | -1 | -1 | | -0.5 | 0 | 1 | 1 | 2 |
| 8 | 5 | -1 | -1 | | -0.5 | 0 | 0 | 0 | 1 |
| 0.04 | Triac 50nM | -2 | 0 | 0 | -2 | -2 | 0 | 0 | 0 |
| | Triac 1uM | -1 | 1 | 0 | -0.5 | -1 | 0.5 | 0.5 | 1 |
| 8 | 5 | -2 | 0 | -0.5 | -2 | -2 | 0 | 0 | 0 |
| 75 | 6 | -2 | -1 | 0 | -2 | -1 | 1 | 0.5 | 1 |
| 0.1 | 7 | -1 | 1 | 0.5 | -1 | -1 | 1 | 1 | 1 |
| 0.08 | 8 | -2 | 1 | 0.5 | -1 | 0.5 | 0.5 | 1 | 1 |

Key to Table 3

| Number | Name | Conc | Ki (Kd) (Nmol/l) |
|---|---|---|---|
| 1 | Trilodothyronine | 1 μM | 0 |
| 2 | Tri-form | 1 μM | 0 |
| 3 | Thyroxine | 1 μM | 10 |
| 4 | KB 131-007 | 1 μM | 20 |
| 5 | SKF 94-901 | 1 μM | 8 |
| 6 | DIMIT | 1 μM | 75 |
| 7 | CIBA 7f* | 1 μM | 0 |
| 8 | KB 131026 | 1 μM | 0 |

(continued)

| Number | Name | Conc | Ki (Kd) (Nmol/l) |
|---|---|---|---|
| | L-T1** | 10 μM | 20000 |

*N-[3,5-Dimethy-4-(4'-hydroxy-3'-isopropylphenoxy)-phenyl]oxamic Acid
**3-idodo-L-Thronine, (Hanning Berlin Germany)

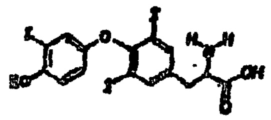

1) Triiodothyronine

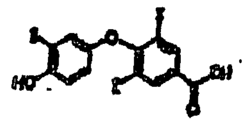

2) Tri-form

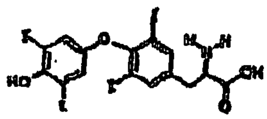

3) Thyroxine

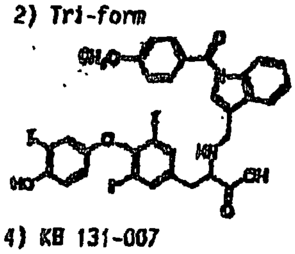

4) KB 131-007

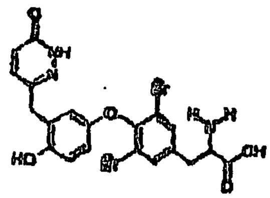

5) SKF 94-901

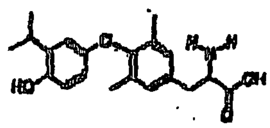

6) DIMIT

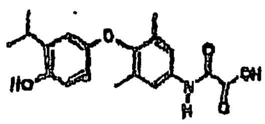

7) CIBA 7f

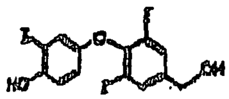

8) KB 131 026

TABLE 4

| Subject | | A | A | B | B | C | C | C |
|---|---|---|---|---|---|---|---|---|
| Transcript | cycles | C | TR 1 | C | TR 1 | C | TR 0.1 | TR 1 |
| GAPDH | 24 | | | | | ND | ND | ND |
| cyclophilin | 20 | | | | | ND | ND | ND |
| Keratin 5 | 19 | | + | | + | | | |
| Keratin 14 | 19 | | + | | + | | | |
| Keratin 1 | 17 | | ++ | | + | | | + |
| Keratin 10 | 19 | | (+) | | (+) | | | |
| TGase-K | 32 | | | | (+) | | + | |
| Involucrin | 25 | | | | | ND | ND | ND |
| RXRθ | 27 | | (+) | | (+) | ND | ND | ND |
| SCCE | 25 | | + | | ++ | | + | + |
| ICAM-1 | 35 | nd | nd | nd | nd | ND | ND | ND |
| IL-6 | 25 | nd | nd | nd | nd | ND | ND | ND |
| 5θ-red.I | 30 | | | | - | ND | ND | ND |

(continued)

| Subject | | A | A | B | B | C | C | C |
|---|---|---|---|---|---|---|---|---|
| 5θ-red. II | 35 | nd | nd | nd | nd | ND | ND | ND |
| Col1A1 | 25 | | ++ | | ++ | | + | + |
| Col3A1 | 22 | | ++ | | + | | + | + |
| Collase | 35 | nd | nd | nd | nd | ND | ND | ND |
| CTGF | 30 | | + | | ++ | | - | - |
| Fibronec | 22 | | + | | + | | | - |
| CRABPII | 25 | | (-) | | - | | | |
| | | | | | | | | |
| nd= not detected, ND= not determined | | | | | | | | |

## Claims

1. A method of cosmetic treatment of skin, which comprises applying to the skin a topical skin treatment composition which contains a pharmacologically acceptable base and Triac in a concentration of less than or equal to 50 mg/100 ml of the composition, in which the composition is applied to skin of so as to act against cosmetic conditions selected from stria, roughened skin, intrinsically aged skin, photodamaged skin, wrinkled skin, collagen-deficient skin and scarred skin such that application of the composition to the skin effects collagen promotion and an improvement in the skin's cosmetic condition without resulting in systemic metabolism of said Triac.

2. A method according to claim 1, in which the concentration is less than or equal to 20mg/100ml.

3. A method according to claim 1 or 2, in which the Triac is in chemically pure form.

4. A method according to any preceding claim, in which the pharmacologically acceptable base is selected from oil-in-water emulsions, sprays, liposomes and solutions.

5. A method according to any preceding claim, in which the Triac is at least partially dissolved in a solvent.

6. A method according to claim 5, in which the solvent is an organic solvent.

7. A method according to claim 5, in which the solvent is alcohol, or an alcohol and water solution.

8. A method according to claim 5, in which the alcohol is ethanol or isopropanol.

9. A method according to any preceding claim, in which the composition contains at least one of a Vitamin D analogue, a glucocorticoid and a retinoic acid receptor binding compound.

10. A method of manufacturing a medicament intended for topical application to the skin for treatment of corticosteroid atrophy so as to effect an improvement in the skin's condition without resulting In systemic metabolism of said medicament, **characterised in that** said medicament comprises a composition containing Triac in a concentration of less than or equal to 50 mg/100 ml of the composition, together with a pharmacologically acceptable base.

11. A method of cosmetic treatment of skin, which method comprises applying to the skin a topical skin treatment composition which contains a pharmacologically acceptable base and either triiodothyronine or a thyroid hormone-like compound, In a concentration of less than or equal to 50 mg/100 ml of the composition, in which said compound binds to thyroid hormone receptor TRα or TRβ with a dissociation constant Kd, lower than 1 μM, wherein

$$Kd = (R) \cdot (L) / (RL),$$

where (R) is the concentration of receptor, (L) is the concentration of ligand, and (RL) is the concentration of the receptor-ligand complex, In which the composition is applied to skin of so as to act against cosmetic conditions selected from stria, roughened skin, intrinsically aged skin, photodamaged skin, wrinkled skin, collagen-deficient skin, and scarred skin, such that application of the composition to the skin effects collagen promotion and an improvement In the skin's cosmetic condition.

**12.** A method according to claim 11, wherein said thyroid hormone-like compound is selected from 3,5,3' tri-iododothyroacetic acid (Triac), 3,5,3' triiodothyroformic acid (Tri-form), N-[3,5-dimethyl-4-(4'-hydroxy-3'-isopropylphenoxy)-phenyl] oxamic acid (CIBA 7f), 5-dimethyl-3'-isopropylthyronine (DIMIT), 4-(4'-hydroxy 3'-iodophenoxy) 3,5 diiodo phenyl ethanol (KB 131-026 as defined herein), and a compound of the following formula

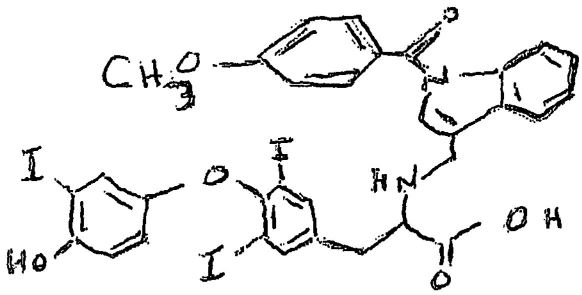

## Patentansprüche

**1.** Verfahren zur kosmetischen Hautbehandlung, welches das Applizieren einer topischen Zusammensetzung zur Hautbehandlung auf die Haut umfasst, welche eine pharmakologisch verträgliche Grundlage und Triac in einer Konzentration von weniger oder gleich 50 mg/100 ml der Zusammensetzung enthält, wobei die Zusammensetzung zur Wirkung gegen kosmetische Zustände auf die Haut appliziert wird, die ausgewählt sind aus Stria, rauher Haut, intrinsisch gealterter Haut, lichtgeschädigter Haut, faltiger Haut, Collagenmangel der Haut und Hautvernarbung, so dass die Applikation der Zusammensetzung auf die Haut eine Collagenförderung und eine Verbesserung des kosmetischen Zustands der Haut bewirkt, ohne zum systemischen Metabolismus der Triac zu führen.

**2.** Verfahren nach Anspruch 1, wobei die Konzentration weniger oder gleich 20 mg/100 ml beträgt.

**3.** Verfahren nach Anspruch 1 oder 2, wobei die Triac in chemisch reiner Form vorliegt.

**4.** Verfahren nach irgendeinem vorhergehenden Anspruch, wobei die pharmakologisch verträgliche Grundlage aus Öl-in-Wasser-Emulsionen, Sprays, Liposomen und

**5.** Verfahren nach irgendeinem vorhergehenden Anspruch, wobei die Triac wenigstens teilweise in einem Lösungsmittel gelöst ist.

**6.** Verfahren nach Anspruch 5, wobei das Lösungsmittel ein organisches Lösungsmittel ist.

**7.** Verfahren nach Anspruch 5, wobei das Lösungsmittel Alkohol ist oder eine Lösung aus Alkohol und Wasser.

**8.** Verfahren nach Anspruch 5, wobei der Alkohol Ethanol oder Isopropanol ist.

**9.** Verfahren nach irgendeinem vorhergehenden Anspruch, wobei die Zusammensetzung wenigstens eines von einen Vitamin D-Analogen, einem Glucocorticoid und einer Retinsäurerezeptor-bindenden Verbindung enthält.

**10.** Verfahren zur Herstellung eines zur topischen Applikation auf die Haut vorgesehenen Medikaments zur Behandlung von Corticosteroidatrophie, um eine Verbesserung des kosmetischen Zustands der Haut zu bewirken, ohne zum systemischen Metabolismus des Medikaments zu führen, **dadurch gekennzeichnet, dass** das Medikament eine Zusammensetzung umfasst, die Triac in einer Konzentration von weniger oder gleich 50 mg/100 ml der Zusammensetzung zusammen mit einer pharmazeutisch verträglichen Grundlage enthält.

**11.** Verfahren zur kosmetischen Hautbehandlung, wobei das Verfahren das Applizieren einer topischen Zusammensetzung zur Hautbehandlung auf die Haut umfasst, die eine pharmakologisch verträgliche Grundlage und entweder Triiodthyronin oder eine Thyroidhormon-ähnliche Verbindung in einer Konzentration von weniger oder gleich 50 mg/100 ml der Zusammensetzung enthält, wobei die Verbindung an Thyroidhormonrezeptor TRα oder TRβ mit einer Dissoziationskonstante, Kd, von weniger als 1 μM bindet, wobei

$$K_d = (R) * (L)/(R/L),$$

wobei (R) die Rezeptorkonzentration, (L) die Ligandenkonzentration und (RL) die Konzentration des Rezeptor-Ligand-Komplexes ist, wobei die Zusammensetzung zur Wirkung gegen kosmetische Zustände auf die Haut appliziert wird, die ausgewählt sind aus Stria, rauher Haut, intrinsisch gealterter Haut, lichtgeschädigter Haut, faltiger Haut, Collagenmangel der Haut und Hautvernarbung, so dass die Applikation der Zusammensetzung auf die Haut eine Collagenförderung und eine Verbesserung des kosmetischen Zustands der Haut bewirkt.

**12.** Verfahren nach Anspruch 11, wobei die Thyroidhormonähnliche Verbindung ausgewählt ist aus 3,5,3'-Triiodthyroessigsäure (Triac), 3,5,3'-Triiodthyroameisensäure (Tri-form), N-[3,5-Dimethyl-4-(4'-hydroxy-3'-isopropylphonoxy)-phenyl]oxaminsäure (CIBA 7f), 5-Dimethyl-3'-isopropylthyronin (DIMIT), 4-(4'-Hydroxy-3'-iodphenoxy)-3,5-diiodphenylethanol (KB 131-026, wie hier definiert) und einer Verbindung der folgenden Formel

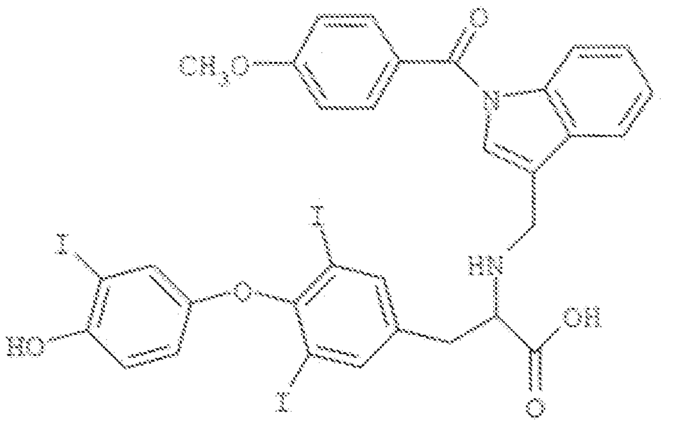

## Revendications

**1.** Procédé de traitement cosmétique de la peau, qui consiste à appliquer sur la peau une composition pour le traitement local de la peau qui contient une base pharmacologiquement acceptable et du Triac en une concentration inférieure ou égale à 50 mg/100 ml de la composition, dans lequel la composition est appliquée sur la peau de manière à agir contre des états cosmétiques choisis parmi des stries, une peau rugueuse, une peau intrinsèquement âgée, une peau photo-endommagée, une peau ridée, une peau déficiente en collagène et une peau cicatrisée de sorte que l'application de la composition sur la peau ait pour effet la promotion du collagène et une amélioration de l'état cosmétique de la peau sans résulter en un métabolisme systémique dudit Triac.

**2.** Procédé selon la revendication 1, dans lequel la concentration est inférieure ou égale à 20 mg/100 ml.

**3.** Procédé selon la revendication 1 ou 2, dans lequel le Triac est sous une forme chimiquement pure.

**4.** Procédé selon l'une quelconque des revendications précédentes, dans lequel la base pharmacologiquement acceptable est choisie parmi les émulsions huile dans l'eau, les aérosols, les liposomes et les solutions.

**5.** Procédé selon l'une quelconque des revendications précédentes, dans lequel le Triac est au moins partiellement

dissous dans un solvant.

**6.** Procédé selon la revendication 5, dans lequel le solvant est un solvant organique.

**7.** Procédé selon la revendication 5, dans lequel le solvant est un alcool, ou une solution hydroalcoolique.

**8.** Procédé selon la revendication 5, dans lequel l'alcool est l'éthanol ou l'isopropanol.

**9.** Procédé selon l'une quelconque des revendications précédentes, dans lequel la composition contient au moins l'un parmi un analogue de la vitamine D, un glucocorticoïde et un composé qui se lie au récepteur de l'acide rétinoïque.

**10.** Procédé de préparation d'un médicament destiné à l'application locale sur la peau pour le traitement d'une atrophie corticostéroïdale ayant pour effet une amélioration de l'état de la peau sans résulter en un métabolisme systémique dudit médicament, **caractérisé en ce que** ledit médicament comprend une composition contenant du Triac en une concentration inférieure ou égale à 50 mg/100 ml de la composition, ainsi qu'une base pharmacologiquement acceptable.

**11.** Procédé de traitement cosmétique de la peau, lequel procédé consiste à appliquer sur la peau une composition pour le traitement local de la peau qui contient une base pharmacologiquement acceptable et la triiodothyronine ou un composé ressemblant à une hormone thyroïdienne, en une concentration inférieure ou égale à 50 mg/100 ml de la composition, où ledit composé se lie au récepteur de l'hormone thyroïdienne TR$\alpha$ ou TR$\beta$ avec une constante de dissociation Kd, inférieure à 1 $\mu$M, où

$$Kd = (R) * (L) / (RL),$$

où (R) est la concentration du récepteur, (L) est la concentration du ligand, et (RL) est la concentration du complexe récepteur-ligand, où la composition est appliquée sur la peau de manière à agir contre des états cosmétiques choisis parmi les stries, une peau rugueuse, une peau intrinsèquement âgée, une peau photo-endommagée, une peau ridée, une peau déficiente en collagène, et une peau cicatrisée, de sorte que l'application de la composition sur la peau ait un effet sur la promotion du collagène et une amélioration de l'état cosmétique de la peau.

**12.** Procédé selon la revendication 11, dans lequel ledit composé ressemblant à une hormone thyroïdienne est choisi parmi l'acide 3,5,3' tri-iododothyroacétique (Triac), l'acide 3,5,3'-triiodothyroformique (Tri-forme), l'acide N-[3,5-diméthyl-4-(4'-hydroxy-3'-isopropylphénoxy)-phényl] oxamique (CIBA 7f), la 5-diméthyl-3'-isopropylthyronine (DIMIT), le 4-(4'-hydroxy-3'-iodophénoxy)-3,5-diiodo phényl éthanol (KB 131-026 tel que défini ici), et un composé de formule suivante

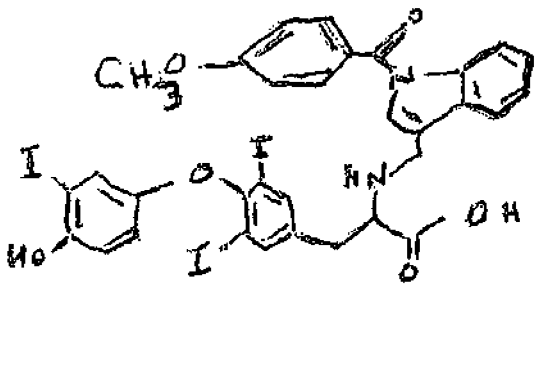

FIG. 1

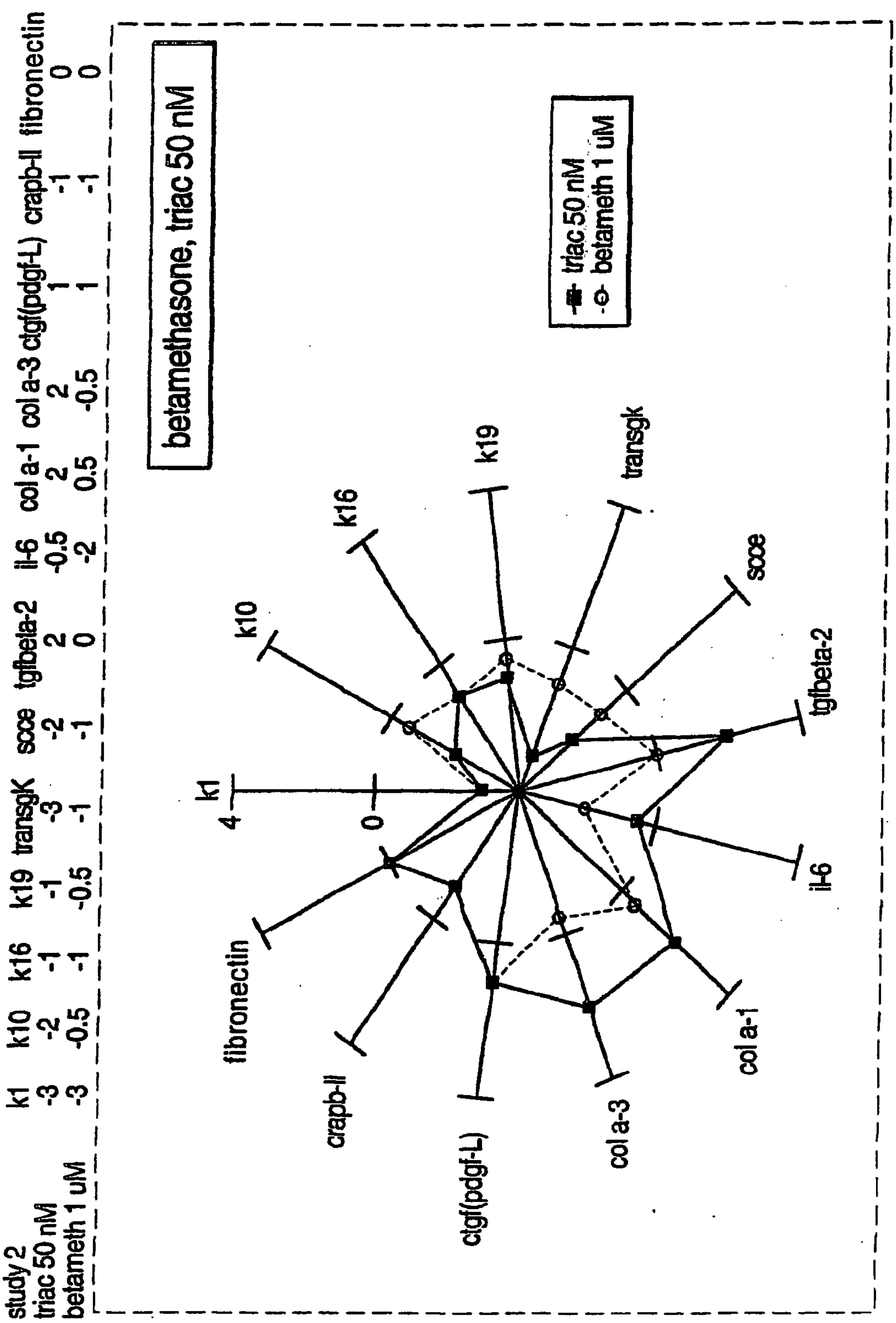
study 2
triac 50 nM
betameth 1 uM
k1 -3 -3
k10 -2 -0.5
k16 -1 -1
k19 -1 -0.5
transgK -3 -1
scce -2 -1
tgfbeta-2 2 0
il-6 -0.5 -2
col a-1 2 0.5
col a-3 2 -0.5
ctgf(pdgf-L) 1 1
crapb-II -1 -1
fibronectin 0 0
betamethasone, triac 50 nM
triac 50 nM
betameth 1 uM
k1
4
0
k10
k16
k19
transgk
scce
tgfbeta-2
il-6
col a-1
col a-3
ctgf(pdgf-L)
crapb-II
fibronectin

FIG. 2

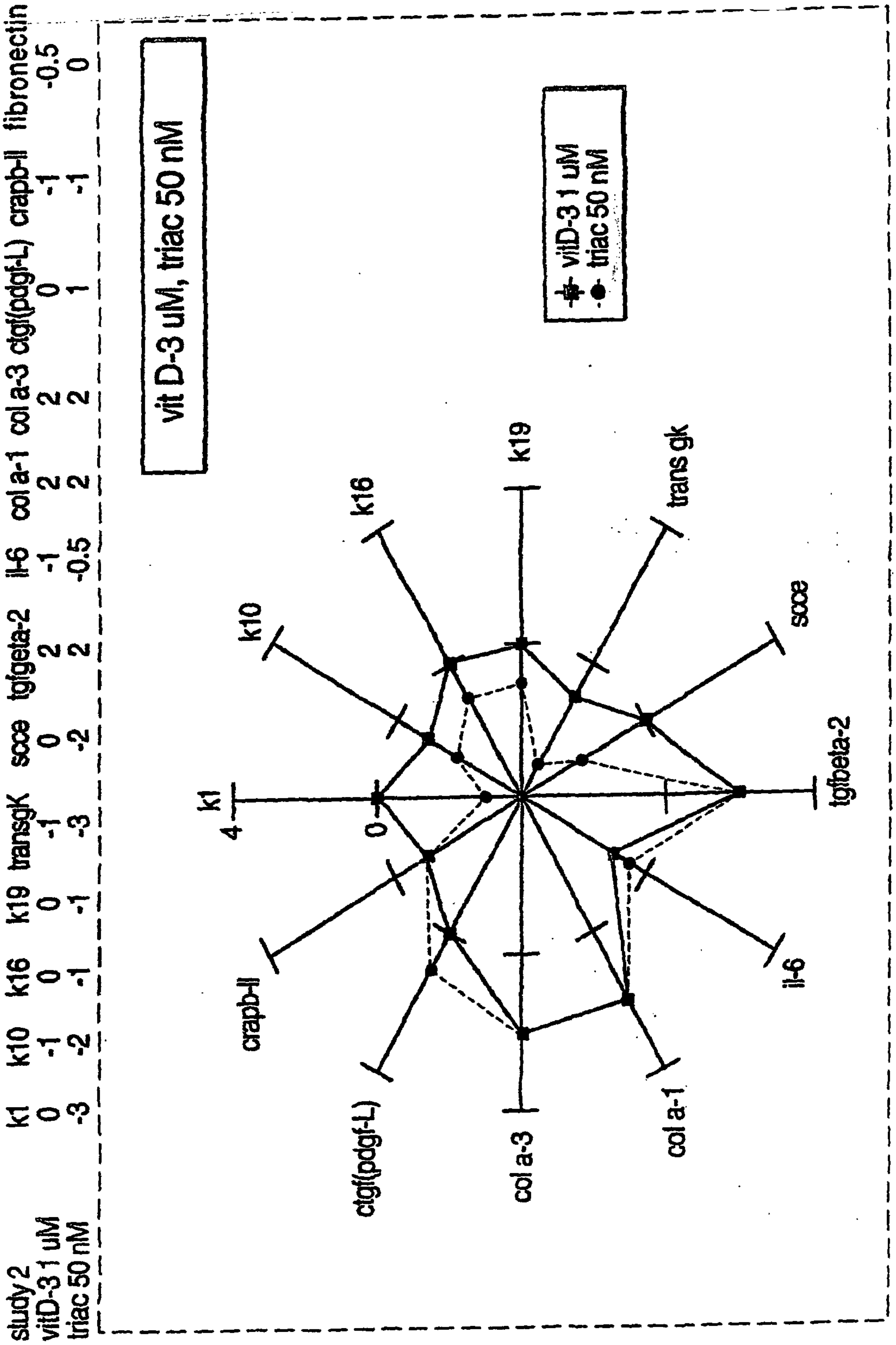
study2 k1 k10 k16 k19 transgK scce tgfgeta-2 il-6 col a-1 col a-3 ctgf(pdgf-L) crapb-II fibronectin
vitD-3 1 uM 0 -1 0 0 -1 0 2 -1 2 2 0 -1 -0.5
triac 50 nM -3 -2 -1 -1 -3 -2 2 -0.5 2 2 1 -1 0
vit D-3 uM, triac 50 nM
vitD-3 1 uM
triac 50 nM
k1
4
0
k10
k16
k19
trans gk
scce
tgfbeta-2
il-6
col a-1
col a-3
ctgf(pdgf-L)
crapb-II

FIG. 3

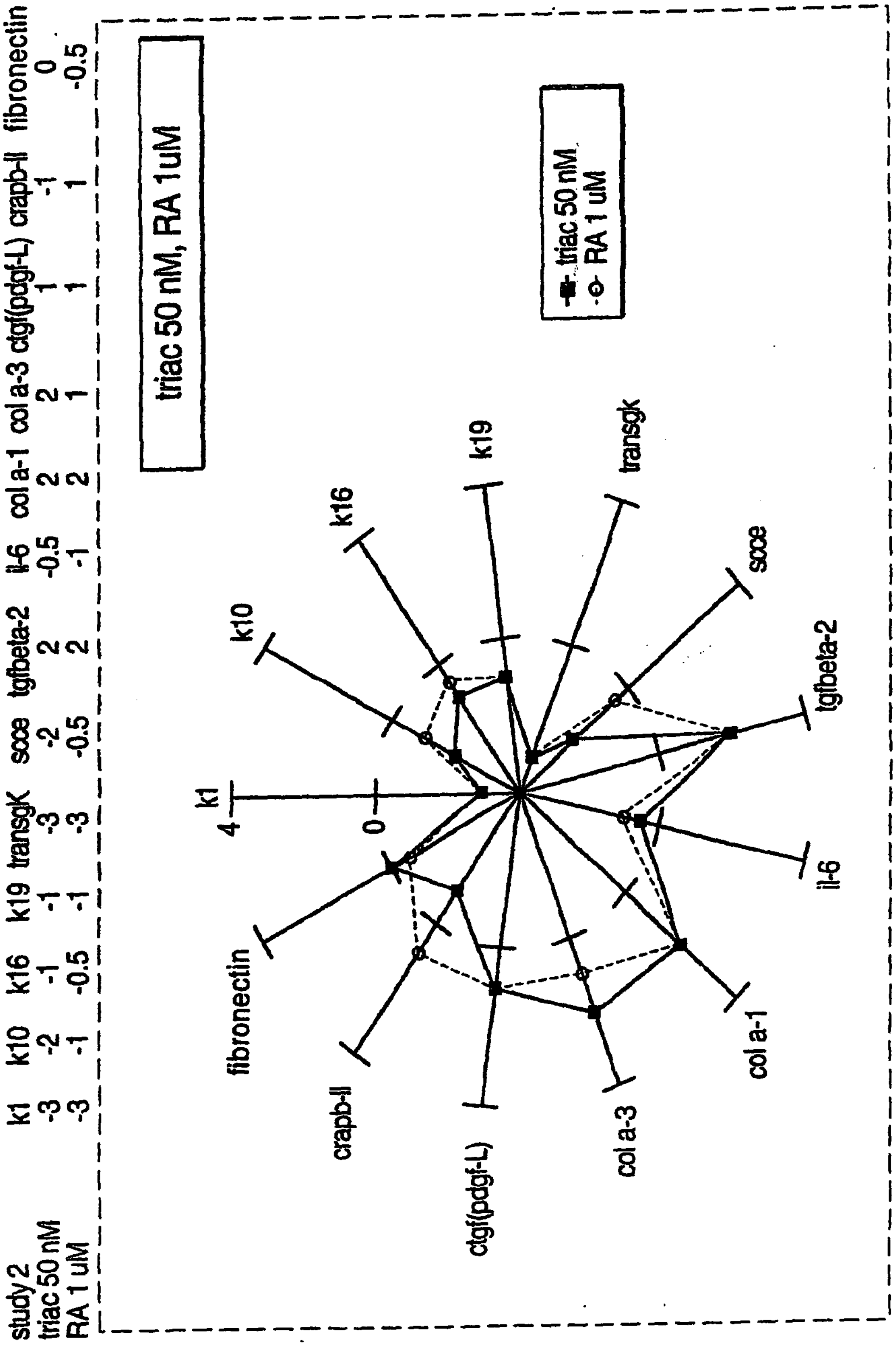
study2
triac 50 nM
RA 1 uM
k1 -3 -3
k10 -2 -1
k16 -1 -0.5
k19 -1 -1
transgK -3 -3
scce -2 -0.5
tgfbeta-2 2 2
il-6 -0.5 -1
col a-1 2 2
col a-3 2 1
ctgf(pdgf-L) 1 1
crapb-II -1 1
fibronectin 0 -0.5
06/05/95
triac 50 nM, RA 1uM
triac 50 nM
RA 1 uM
k1
4
0
k10
k16
k19
transgk
scce
tgfbeta-2
il-6
col a-1
col a-3
ctgf(pdgf-L)
crapb-II
fibronectin

FIG. 4

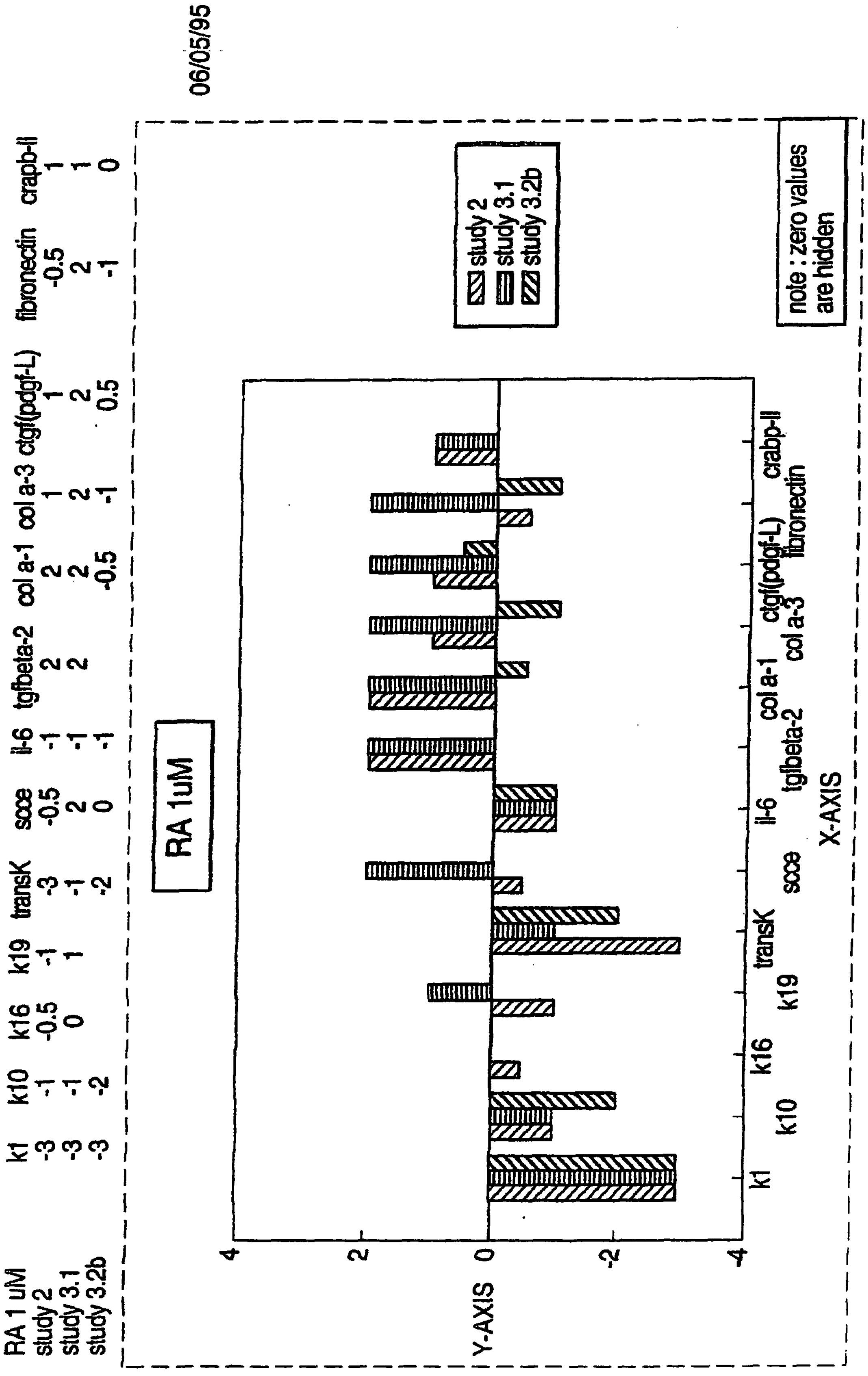
RA 1 uM
study 2
study 3.1
study 3.2b
k1 -3 -3 -3
k10 -1 -1 -2
k16 -0.5 0
k19 -1 1
transK -3 -1 -2
scce -0.5 2 0
il-6 -1 -1 -1
tgfbeta-2 2 2
col a-1 2 2 -0.5
col a-3 1 2 -1
ctgf(pdgf-L) 1 2 0.5
fibronectin -0.5 2 -1
crapb-II 1 1 0
06/05/95
RA 1uM
study 2
study 3.1
study 3.2b
note : zero values are hidden
Y-AXIS
4
2
0
-2
-4
k1
k10
k16
k19
transK
scce
il-6
tgfbeta-2
col a-1
col a-3
ctgf(pdgf-L)
fibronectin
crabp-II
X-AXIS

*FIG. 5*

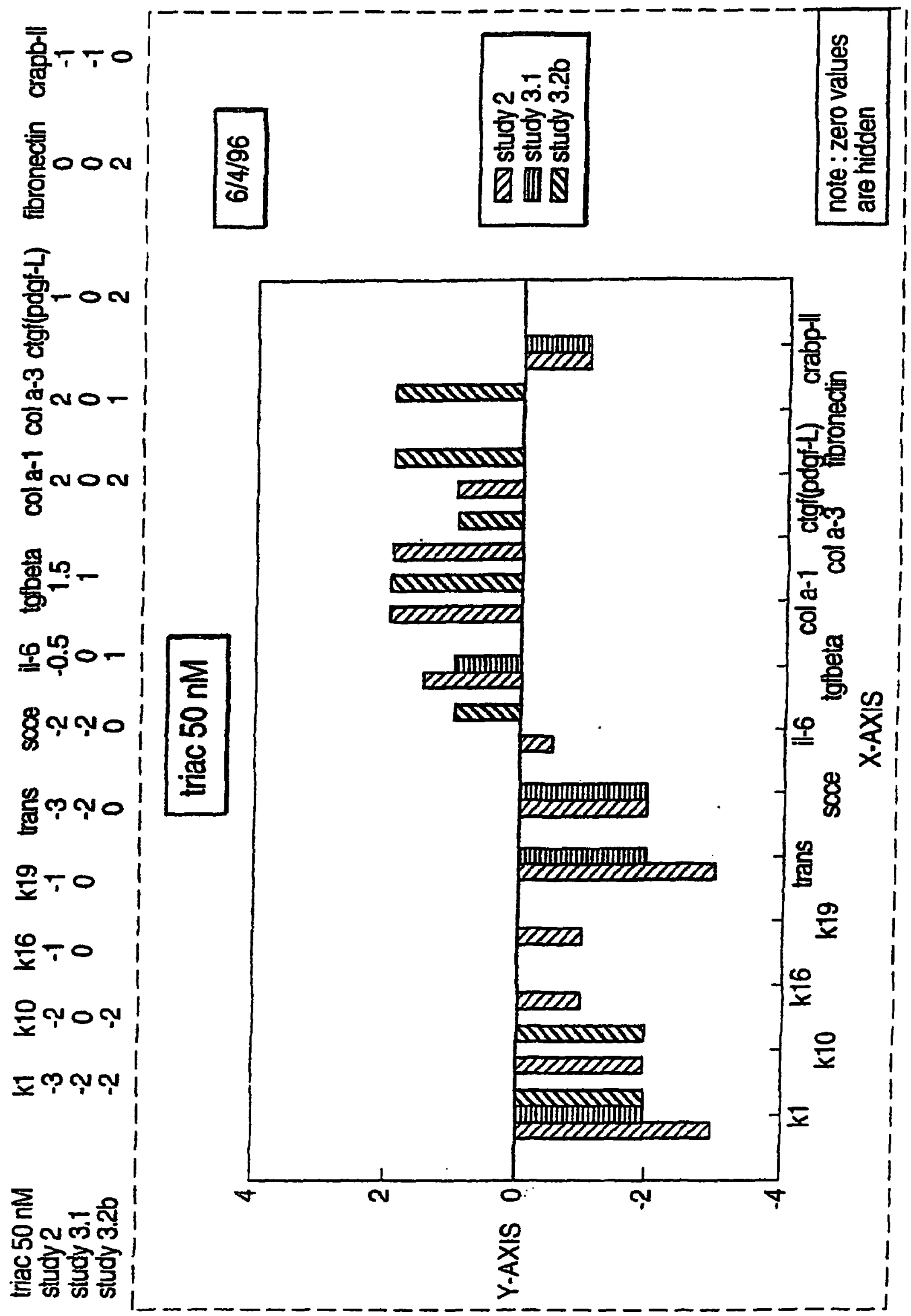
triac 50 nM k1 k10 k16 k19 trans scce il-6 tgfbeta col a-1 col a-3 ctgf(pdgf-L) fibronectin crabp-II
study 2 -3 -2 -1 -1 -3 -2 -0.5 1.5 2 2 1 0 -1
study 3.1 -2 0 0 0 -2 -2 0 1 0 0 0 0 -1
study 3.2b -2 -2 0 0 1 2 1 2 2 0
triac 50 nM
6/4/96
study 2
study 3.1
study 3.2b
note : zero values are hidden
Y-AXIS
4
2
0
-2
-4
k1
k10
k16
k19
trans
scce
il-6
tgfbeta
col a-1
col a-3
ctgf(pdgf-L)
fibronectin
crabp-II
X-AXIS

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5401772 A
- FR 2153202
- FR 2197577
- EP 060776 A
- CH 642851
- GB 1354263 A
- GB 1400851 A
- FR 2354101
- FR 96171
- GB 782745 A
- GB 859546 A
- US 3198702 A
- US 5284971 A
- US 3649679 A
- US 3357887 A
- US 412579 A
- US 4168385 A
- US 5179097 A
- EP 0580550 A
- EP 018351 A
- EP 00465343 A
- GB 7822745 A


### Non-patent literature cited in the description


- *Journal of Medicinal Chemistry,* 1963, vol. 6, 554-563
- **H. A. SELENKOW ; S. P. ASPER.** *Jr., Physiol. Rev.,* 1955, vol. 35, 426
- **C. S. PITMAN ; J. A. PITMAN.** In Handbook of Physiology, Section 7: Endocrinology. American Physiological Society, 1974, vol. 3, 233
- **E. C. JORGENSEN.** *Pharm. Ther. B,* 1976, vol. 2, 661
- **HERMENS W. A. J.** J Pharmaceutisch Weekblad Scientific. 1992, vol. 14
- *Febs Letter,* 1989, vol. 258, 35-38
- *J. Invest . Derm.,* vol. 90, 472
- **FUCHS E.** *J. Cell Biol.,* vol. 111, 2807
- **EGULRUD et al.** *ACTA DERM Venereol,* 1993, vol. 73, 181-184
- **SONDELL et al.** *J. Invest Derm,* 1995, 819-823
- **WEST et al.** *J. Invest. Derm.,* 1992, vol. 1, 95
- *J.Cell Biol,* 1987, vol. 105, 427-440
- **GRIFFITHS et al.** *Br J Derm,* 1992, vol. 127 (4), 21
- **GRIFFITHS et al.** *Br. J. Derm,* 1992, vol. 127 (4), 21
- **DE RYCKER et al.** *FEBS Lett,* 1984, vol. 174, 34-37
- **RHEINS et al.** *Toxic, in Vitro,* 1994, vol. 8 (5), 1007-1014
- **SAMUELS, H. et al.** *Endocrinology,* 1979, vol. 105, 80-85
- **TALWAR et al.** *J. Inv. Derm,* 1995, vol. 105, 285-290
- Mechanisms of Thyroid Hormone action. **LAVIN, T.N.** In the textbook of Endocrinology. W. B. Saunders, 1989
- **DROZDZM M et al.** *Endokrinologie,* February 1979, vol. 73 (1), 105-11
- **MURATA Y et al.** *J Clin Endocrinol Metab,* February 1987, vol. 64 (2), 334-9
- **WATXKE, H. et al.** *Thrombosis Research,* 15 April 1987, vol. 46 (2), 347-53
- **MURATA Y et al.** *JCEM,* 1983, vol. 57, 1233-1239
- **LEE J et al.** *Endocrinology,* 1992, vol. 130, 2733-2738
- **CECCARELLI, P et al.** *JCEM,* 1987, vol. 65, 242-246
- **TOMIO-CANIC M et al.** *Journal of Investigative Dermatology,* 1992, vol. 99, 842-847
- **BLUMENBERG M et al.** *Invest Dermatol,* June 1992, vol. 98 (6), 42-49
- **OHTSUKI M et al.** *J Dermatol,* 19 November 1992, 774-80
- **HOLT, P.J.A et al.** *Journal of Dermatology,* 1976, vol. 95, 513-518
- **CRONRATH CM et al.** *Endocrinology,* January 1987, vol. 120 (1), 43-8
- **TOWLE H. C ; MARIASH C. N.** *Federation Proceedings,* August 1986, vol. 45 (9), 2406-11
- **LEMMEN C. et al.** *Biological Chemistry Hoppe-Seyler,* 1986, vol. 367 (7), 533-537
- **AMOROSA L. F et al.** *Biochim Biophys Acta,* 09 February 1984, vol. 792 (2), 192-8
- **ARMER P et al.** *Diabetes,* 1984, vol. 33 (4), 369-375

- *Coronary Dru Project, JAMA,* 1972, vol. 220, 996-1008
- **AKESSON, A et al.** *Acta Radiologica Diagnosis,* vol. 27, 91-94
- **HUNT, T et al.** *Annals Surgery,* 1969, vol. 170, 633-641
- **VARANI, J et al.** *J. Invest Dermatol,* 1990, vol. 94, 717-723
- **LOIREAU A et al.** *Biochem Pharmacol,* 1986, vol. 35, 1691-1696
- **MOLONEY S. J et al.** *Photochem Photobiol,* 1992, vol. 56, 505-511
- **SCHWARTZ E et al.** *J. Investl. Dermatol,* 1994, vol. 102, 241-246
- **GRIFFITHS C. E. M et al.** *NEJM,* 1993, vol. 329, 530-535
- **ROTI, E et al.** *Endocrine Reviews,* 1993, vol. 14, 401-423
- **LEV-RAN.** *A Perspectives in Biology & Medicine,* 1994, vol. 37, 486-494
- **APRILETTI J. et al.** *J. Biol. Chem,* 1988, vol. 263, 9409-9417